# EUROPEAN PATENT SPECIFICATION

(11) **EP 4 340 903 B1**
(45) Date of publication and mention of the grant of the patent: **23.07.2025**
(21) Application number: 22726305.0
(22) Date of filing: 13.05.2022
(51) Int. Cl.: A61L 27/44

(54) **SYSTEM FOR SELECTIVE THERMAL INHIBITION OF THE ACTIVITY OF NERVES AND NEURONAL STRUCTURES**
SYSTEM ZUR SELEKTIVEN THERMISCHEN HEMMUNG DER AKTIVITÄT VON NERVEN UND NEURONALEN STRUKTUREN
SYSTÈME D'INHIBITION THERMIQUE SÉLECTIVE DE L'ACTIVITÉ DE NERFS ET DE STRUCTURES NEURONALES

(30) Priority: 18.05.2021 IT 202100012785
(43) Date of publication of application: 27.03.2024
(73) Proprietor: Scuola Superiore di Studi Universitari e di Perfezionamento Sant'Anna, 56127 Pisa (IT)
(72) Inventor: AGNESI, Filippo, 20134 Milano (IT); MICERA, Silvestro, 50132 Firenze (IT); REDOLFI RIVA, Eugenio, 56124 Pisa (IT)
(74) Representative: Di Giovine, Paolo
(86) International application number: PCT/IB2022/054483
(87) International publication number: WO 2022/243823

(56) References cited:
- EP-A1- 3 299 040
- US-A1- 2013 310 908
- US-A1- 2019 357 847
- MUNSHI RAHUL ET AL: "Magnetothermal genetic deep brain stimulation of motor behaviors in awake, freely moving mice", ELIFE, vol. 6, 15 August 2017 (2017-08-15), pages 1 - 26, XP055883903, DOI: 10.7554/eLife.27069

## Description

### Field of the invention

The present invention relates in general to the field of biomedical devices, and more precisely relates to a system for slowing, inhibiting or blocking the activity of nerves and neuronal structures, useful for research purposes and for medical use by means of surgical implantation in patients in need thereof, for example in the treatment of pain, in the treatment of muscle spasms and epilepsy, and more generally in the treatment of those neurological conditions that may benefit from the modulation of the neuronal activity through slowing, inhibiting or blocking. Such neuromodulation can have a transient blocking effect on nerve activity or can restore the proper functioning of nerve structures affected by hyperactivity.

### Background of the Invention

Neuromodulation is a field of medicine that consists of altering the physiological, or patho-physiological, neuronal activity for the purpose of treating medically relevant conditions. At least in a first approximation, neuromodulation can be characterised by activation or inhibition of the neuronal substrate concerned, and can for instance be achieved by applying appropriate chemical compounds to the structure to be modulated. However, this approach is fraught with a number of problems and, for clinical applications, it has now been superseded by neuromodulation carried out by electrical stimulation. This second approach has the advantage that neurons already use the movement of ions, electrically charged chemical species, to transmit information; therefore, neurons are also easily activated by rapid changes in an electric field.

Neuromodulation of a nerve by electrical stimulation generally consists of implanting an electrode around the nerve so that the exposed metal surfaces of the electrode face the nerve and wrap around it; this is why such electrodes are also called *cuff electrodes.* Neuromodulation can also be performed by using an electrode that penetrates the tissue and reaches the individual fascicles within the nerve, which condition is useful for increasing the selectivity of neuromodulation, at the expense, however, of the invasiveness of the implantation procedure and an increased inflammatory response to a foreign body; such electrodes are called *intraneural.* The technique of neuromodulation by electrical stimulation on the one hand represents a successful technique, useful for treating a variety of conditions, but on the other hand it is unable to generate neuronal substrate inhibition or block the conduction of neuronal signals along nerves. There is also a technique called Kilohertz Frequency Alternating Current (KHFAC), which can use electrical current to block the conduction of neuronal signals. However, this technique is burdened by several technical complications that have made it unworkable in clinical practice, although potentially effective. EP 3 299 040 A1 discloses macroscopically alignable, injectable, soft hydrogel composition comprising soft, anisometric magnetoceptive microgels comprising magnetic particles, particularly superparamagnetic iron oxide nanoparticles, and a crosslinkable biocompatible matrix hydrogel composition. US 2013/310908 A1 discloses silk fibroin-based photothermal elements comprising a plurality of plasmonic nanoparticle distributed in a silk fibroin matrix, which can be used as an implantable and biodegradable heating element activated by light. MUNSHI RAHUL ET AL, ELIFE, vol. 6, 15 August 2017 (2017-08-15), pages 1-26 (XP055883903) discloses magnetothermal genetic stimulation of awake mice, providing tetherless deep brain activation sufficient to evoke motor behavior.

To date, to the best of the Applicant's knowledge, there are no other approaches to inhibiting the activity of a neuronal structure or the conduction of neuronal activation along a nerve, let alone already developed commercial devices that are able to carry out such treatments with proven utility in the medical field. Therefore, the need for technically effective devices that can be used in clinical practice is strongly felt in the industry.

### Summary of the invention

Now the inventors have found a system capable of acting thermally on a nerve or on a neuronal structure in general, in order to slow, inhibit or temporarily block the neuronal activity thereof thanks to a controlled and localised rise in temperature, obtained by converting near-infrared light radiations into thermal energy.

The localisation of this controlled heat development effect, precisely at the nerve tissue to be treated, was realised by the inventors thanks to the use of a matrix that is injectable in the nerve or in the neuronal structure to be treated or on their surface, which is additionally loaded with plasmonic nanoparticles.

The matrix incorporates the nanoparticles within it, controlling their concentration during and after the positioning of the system at the target neuronal structure, preventing the dispersion of the nanoparticles out of the target zone and thus also possible long-term toxic effects related to the internalisation of the nanoparticles in the surrounding cells, while at the same time ensuring a reproducible and controllable heating effect.

This technical solution found by the inventors also facilitates and eases the spatial distribution of the nanoparticles during and after the implantation procedure, thus making the positioning of the nanoparticle matrix controllable. In particular, the matrix comprises a suspension of microbeads loaded with nanoparticles, where the microbeads are globular, sphere-like polymeric structures with sizes ranging from 30 µm to 300 µm, i.e. larger than a cell, but large enough for them to penetrate the nerve and diffuse into the extracellular matrix. This size range also prevents their internalisation within the cell cytoplasm, which is namely an unfavourable condition that might lead to the onset of long-term toxic effects.

An object of the invention is therefore a system for thermally slowing, inhibiting or temporarily blocking the activity of nerves and neuronal structures, comprising an injectable or implantable matrix, loaded with nanoparticles, wherein said matrix loaded with nanoparticles comprises a liquid suspension of polymeric microbeads loaded with nanoparticles and an external source of energy, the essential characteristics of which are defined in the first of the appended claims. Further important features of the system according to the invention are defined in the claims dependent on the first one.

A further object of this invention is the use of the above-mentioned system for research purposes, to slow, inhibit or block *ex vivo* the activity of a neuronal structure previously taken from a subject, for neuroscientific research purposes. The references to methods of treatment in this description are to be interpreted as references to the system of the present invention for use in a method for treatment of the human (or animal) body by therapy.

Further described is a method for slowing, inhibiting or blocking the activity of nerves or neuronal structures in a subject in need thereof, comprising the steps of:
i) providing the aforesaid system comprising an injectable or implantable matrix, loaded with plasmonic or superparamagnetic nanoparticles, and an external source of NIR radiations or a source of alternate magnetic field, respectively;
ii) injecting the matrix loaded with the nanoparticles in the neuronal structure to be treated;
iii) placing the external energy source in a position adjacent to the neuronal structure to be treated;
iv) activating the source for energy emission long enough to slow, inhibit or block the neuronal activity in the structure.

Other important features of the system, use and method according to the invention are given in the following detailed description, also with reference to the figures.

### Brief description of the figures

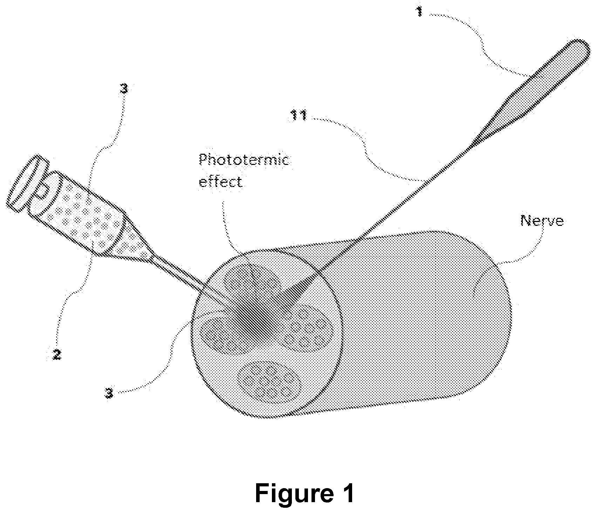

Figure 1: schematic representation of a first embodiment of the system of the invention in which a matrix of microbeads loaded with plasmonic nanoparticles and suspended in a fluid medium is injected in a nerve via syringe, and irradiated with an external NIR laser source;

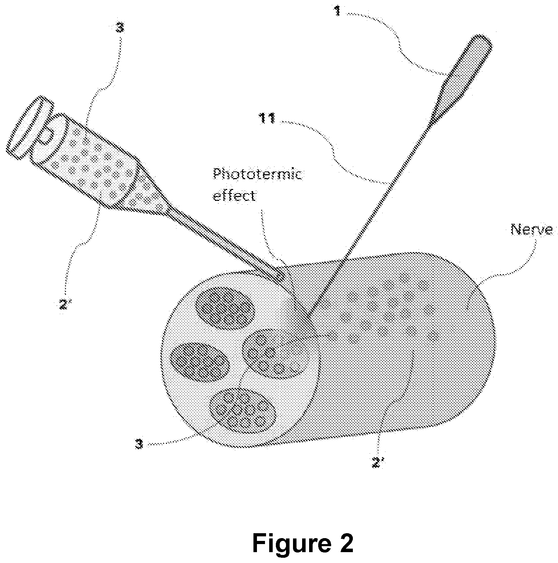

Figure 2: schematic representation of a second embodiment of the system of the invention in which the suspension of microbeads loaded with plasmonic nanoparticles, is injected in a nerve via syringe so as to distribute the matrix over a surface area thereof (Fig. 2a), by suspending these microbeads inside an adhesive material for biological tissues, and irradiated with an external NIR laser source;

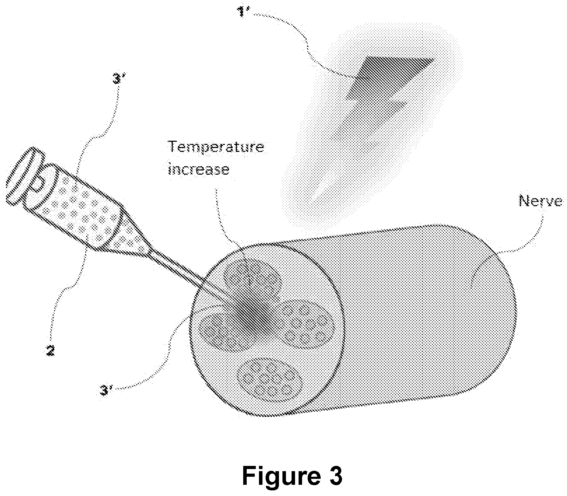

Figure 3: schematic representation of a first embodiment of a system in which a matrix of microbeads loaded with superparamagnetic nanoparticles and suspended in a fluid medium, is injected in a nerve via syringe, and irradiated with an external radio frequency source;

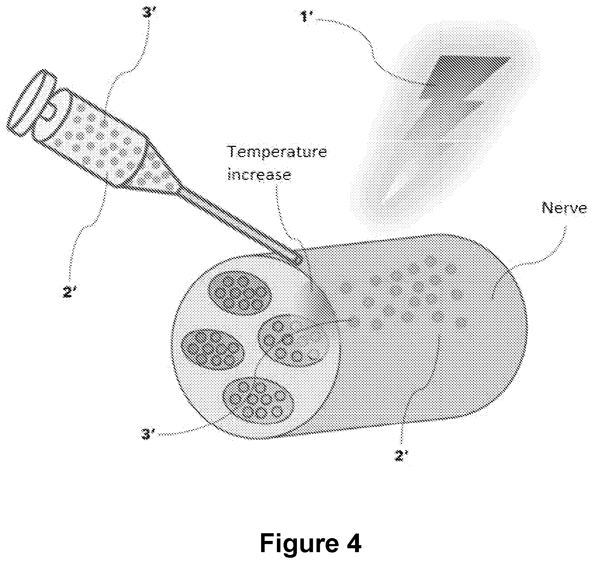

Figure 4: schematic representation of a second embodiment a system in which a fluid matrix loaded with superparamagnetic nanoparticles is injected in a nerve via syringe so as to distribute the matrix over a surface area thereof (Fig. 5a) by suspending these microbeads inside an adhesive material for biological tissues, and irradiated with an external radio frequency source;

### Detailed description of the invention

### Definitions

Within the scope of the present invention, the term "neuronal structure" means any potential target of the present system and method of treatment, forming part of the nervous system; non-limiting examples of neuronal structures according to the invention are field receptors, sensorimotor nerves, autonomic nerves, nerve plexuses, root ganglia of the spinal cord, autonomic ganglia, spinal cord, brain nuclei, brain tracts and cortical structures.

The term "microbeads" is used here to refer to beads, particles or spheres with micrometre sizes, suitable for incorporating the nanoparticles of the system of this invention, which can be obtained by processes known in the state of the art. The diameter of these microbeads is typically between 1 and 1000 µm.

The term "nanoparticles" is used here to refer to particles with nanometric sizes, with a diameter typically between 1 and 100 nm, and also having any shape, e.g. nanospheres, nanocylinders, nanotubes, nanostars and even more sophisticated shapes, which can be obtained according to processes known in the state of the art.

### Detailed description

As mentioned above, this invention relates primarily to a system for thermally slowing, inhibiting or blocking the activity of a neuronal structure, comprising:
- a matrix injectable in the neuronal structure to be treated, or on the surface thereof, loaded with plasmonic nanoparticles; and
- a NIR laser source targeting the aforesaid matrix loaded with plasmonic nanoparticles, in which said matrix is represented by polymeric microbeads. The latter are loaded with plasmonic nanoparticles and suspended in aqueous solution to be thus injected in the target structure.

In such a system, the thermal energy is produced locally by the plasmonic nanoparticles, which can convert the NIR radiations of the external source into thermal energy. After positioning the matrix containing the nanoparticles in the neuronal structure to be treated, the present system ensures that the conversion of the energy absorbed by the nanoparticles into thermal energy produces a localised and controlled rise in temperature for a targeted action on the target neuronal structure, thus minimising the risk of damaging surrounding tissues that do not require treatment. Furthermore, it is noteworthy that the system of this invention is not based on a local temperature rise thanks to a local application of an external heat source to the tissue to be treated, but the heat is produced from within or on the surface of the structure, using an external energy source that is absorbed by the implanted component alone while the tissues are transparent to this source. In this way, it is possible to precisely and selectively control the rise in temperature, in order not only to achieve the desired neuromodulatory effect, but also to limit the exposure of unaffected tissues to heat.

According to the present invention, microbeads loaded with nanoparticles are in injectable form, which can be positioned within the neuronal structure to be treated by simple injection with a special syringe, without the need for resorting to implantation surgery. In this aspect of the invention, the concentration of microbeads to be injected will have to be determined according to the size of the nerve to be treated and the internal fascicles thereof, so that they can diffuse freely within the tissue structure.

Disclosed but not claimed is an aspect where the external source is a source of alternate magnetic fields and the nanoparticles are superparamagnetic nanoparticles, they have diameters of less than 50 nm, e.g. less than 30 nm, or less than 25 nm.

For the realisation of an injectable matrix useful in the system of the invention, use can made of microbeads, whose sizes, for example, are between 1 and 1000 µm, preferably between 30 and 300 µm, which encapsulate the nanoparticles and can be suspended in a fluid injection medium, such as physiological saline solution. Optionally, the injection medium may comprise one or more additional compounds selected from anti-inflammatory agents, adjuvants in wound repair, antibiotics, contrast agents or radioopaque agents.

Following injection, the injection medium is absorbed by the tissues and the microbeads remain trapped at the injection site as they are too large to be removed in the course of biological processes. Depending on the material with which the microbead is made, its residence time in the nerve structure may be longer or shorter, and thanks to its placement by injection, even very small and difficult-to-reach regions in the neuronal structures, such as the single nerve fascicle or a small fraction of a brain nucleus, can thus be easily reached, ensuring a uniform and rapid diffusion of heat within the structure targeted by the treatment.

In one aspect of the invention, the microbeads encapsulating the nanoparticles can be advantageously coated with anti-inflammatory agents and/or with agents capable of reducing foreign-body reactions.

Non-limiting examples of materials suitable for realising microbeads as matrices for the present system are elastomers, such as silicone, polyurethanes, polydimethylsiloxane (PDMS), polyhydroxyalkanoates, preferably including P4HB and P3HB-co-4HB poly(ethylene-vinyl acetate) (PEVA); elastic natural proteins such as collagen, elastin, titin, fibrillin, abductin, silk protein, resilin; thermoresponsive gels, such as polyoxamers; commercial formulations for drug delivery, such as Eudragit^{®}, a class of polymethacrylate-based copolymers that includes neutral, anionic or cationic copolymers based on methacrylic acid and methacrylic/acrylic esters or derivatives thereof; thermoplastic polyesters such as polycaprolactone (PCL), polylactic acid (PLA), polyglycolic acid (PGA) and copolymers thereof; thermosetting materials such as epoxy resins and polyimide; and lipid materials such as waxes (beeswax, carnauba wax, jojoba wax or cetyl palmitate), fatty acids (oleic acid, stearic acid, palmitic acid, alpha-linoleic acid) or phospholipids (phosphatidylcholine or phosphatidylethanolamine). Mixtures of the aforesaid materials are included in the present invention, as well as the addition to the above materials of other polymers such as polymers having the function of stabilising, emulsifying or thickening agents.

The method for encapsulating nanoparticles in these microbeads can be any known microencapsulation technique, including emulsion, nanoprecipitation, coacervation, sol-gel transition, ionotropic gelation or lyophilisation. Microfluidic devices can eventually be used in the preparations and, once the microbeads with the nanoparticles inside have been formed, any washing and purification, and/or sterilisation steps can be carried out.

In order to achieve anchorage to the surface of the nerve structure, the microbeads can be dispersed within a material that can cross-link *in situ.*

In another aspect of the invention, the microbeads loaded with nanoparticles can be dispersed within an adhesive and biocompatible material that, once injected and placed in the neuronal structure to be treated, can be cured, i.e. solidified by conforming it to the surrounding tissues, with a chemical or physical reaction. With this type of matrix, for example, structures suitable for carrying out the method of this invention can be created, for example *cuff-like* structures, completely or partially wrapped around a nerve, or structures of other conceivable shapes that can allow them to be anchored to the surface of the nerve to be treated. The adhesion of the microbeads on the surface of the target neuronal structure allows them to be positioned at the nerve fascicles immediately next to the surface of the neuronal structure to be treated. This takes advantage of the anchorage of the microbeads to the surface of the epineurium (the layer of connective tissue that forms the outer layer of the peripheral nerves) thanks to the presence of the adhesive suspension medium, so as to avoid an invasive operation in those cases in which the nerve fascicles of interest do not require the injection of the microbeads inside the nerve to be reached. In this way, once the microbeads are anchored to the nerve surface and are subjected to heating by external radiation, the heat generated spreads to the underlying nerve structures, acting only on certain superficial fascicles.

Suitable polymers for realising such an adhesive suspending medium are selected from examples in the group consisting of medical resins, polyoxyethylene, protein glues, e.g. fibrin-based glue, such as the commercial glue TISSEL^{®}, cyanoacrylate surgical glues such as n-butyl-2-cyanoacrylate or 2-octyl-cyanoacrylate, and mixtures thereof. Such glues or resins can facilitate *in vivo* cell adhesion of fibroblasts and stem cells thanks to the formation of a polymer network at the injection site. Collagen, hyaluronic acid and other biocompatible proteins can be added to the matrix of the system of the invention in order to increase the integration thereof with the tissue, as well as also excipients, such as glycerol or surfactants, to modify the viscosity of the solution and delay the cross-linking process. In this regard, adhesive suspending media that cross-link *in vivo* by mixing two components (monomer and cross-linking agent) can disperse the microbeads within them simply by adding them to one of the two aforementioned elements to be inserted into a commercial two-component syringe that allows them to be mixed during injection. It will be necessary to vary the concentrations and volumes of the crosslinker and monomer solutions in order to vary the viscosity, injectability and crosslinking time of the adhesive material, so as to allow an efficient anchorage to the nerve surface, or to allow the microbeads to be anchored to structures of different size. In one aspect of the invention, the matrix of the present system may comprise radiopaque compounds to enhance the visibility of the matrix in X-ray analysis or computed tomography techniques; alternatively, the matrix of the present system may comprise air bubbles included so as to enhance visibility in ultrasound *imaging* techniques.

Advantageously, in one aspect of the system of the invention, it further comprises at least one device for measuring the temperature in the neuronal structure, for example a miniaturised device injectable or implantable with the matrix. Optionally, the system of the invention further comprises a control unit connected to the temperature measuring device, which monitors the measured temperature, and adjusts the intensity of the emissions of the source accordingly so as to avoid excessive heating of the tissues and their consequent damage.

In a particular embodiment of the system of this invention, it is the same device for injecting the matrix that comprises a temperature measuring element, for example a thermocouple placed at the tip of the syringe needle. Instead of being immediately retracted, the needle can be held in place after injecting the matrix, and used to measure the temperature and calibrate the external energy source to obtain the correct heat development at the neuronal structure to be treated.

With regard to the nanoparticles, which represent in the system of the invention the active element for converting the energy emitted by an external source into heat, they are plasmonic nanoparticles, selected for example from plasmonic nanoparticles of noble metals such as gold, silver, palladium and platinum; further disclosed but not claimed are superparamagnetic nanoparticles, such as nanoparticles of iron oxide. The heat produced by conversion then diffuses towards the target tissues by flowing through the matrix in which the nanoparticles are immersed. The matrix in this respect has only a passive function, to control the placement of the nanoparticles, their distribution, concentration and to prevent their dispersion out of the target tissue.

According to the invention, the present system comprises at least one NIR laser source of near-infrared light radiations, typically with a wavelength between 700 and 900 nm, and plasmonic nanoparticles that, once exposed to this light source, exhibit a significant light absorption at a particular band of the light spectrum, which is rapidly converted into heat and diffused to the surrounding medium. The use of a NIR source in the present system ensures a consistent penetration of light radiation into the biological tissues, up to a few centimetres, without causing damage to the tissues, as it is not absorbed by them. In addition, the present system is safe and easily modulated thanks to the possibility of controlling the power of the source emission and the emission mode, whether pulsed or continuous, and the fineness and shape of the nanoparticles included in the matrix. Any expert in the field with ordinary knowledge will be able to effortlessly select the characteristics of the nanoparticles, in particular shape and size, as well as the light source and its relative parameters, to modulate the photothermal effect within the matrix on the neuronal structure.

In one aspect of the invention, the system may comprise several NIR sources all emitting at the same wavelength or emitting at different wavelengths. In another aspect of the invention, the one or more NIR sources may emit, all or independently the one of the other, radiations in a continuous or pulsed manner with intensity and shape of the emitted signals that can be modulated according to the specific requirements of the treatment. In one aspect of this invention, the presence of several external energy sources can be combined with the simultaneous use of several matrices each loaded with plasmonic nanoparticles, which absorb different wavelengths, injected or implanted at different points of the target structure. In such embodiments, by changing the light radiation emitted by the external source, it will be possible to change the heating site of the target tissue, without changing the position of the emission source. A more eclectic and selective system can thus be realised.

Sources of alternate magnetic fields suitable for realising the present system may be, for example, sources emitting at one or multiple frequencies between 10 and 300 KHz. Magnetic field oscillations can be produced in a continuous mode or in pulses of determined lengths at specific regular or irregular intervals, with constant or time-varying intensity in a predetermined manner or varied in real time.

In one aspect of the invention, the NIR source may be wearable by the patient, included in any wearable system such as those already on the market.

Figure 1 schematically illustrates a system of the invention comprising a NIR laser source 1 which emits radiation 11 at a nerve to be treated, in which an aqueous solution 2 in which polymeric microbeads containing plasmonic nanoparticles 3 are dispersed have been injected by syringe, thus producing a photoelectric effect at the tissues to be treated. Figure 2 also schematically depicts a system of the invention of this type, in which, however, the microbeads are dispersed in a biocompatible adhesive for tissues 2'. Figures 3-4 depict corresponding systems in which the NIR laser source 1 is replaced by a source of oscillating magnetic field 1', and the microbeads are loaded with superparamagnetic nanoparticles 3' capable of absorbing the radiofrequencies of the external field by producing a temperature increase.

From the attached figures, it is evident how the present system can be adaptable to any conformation and size of the structure to be treated; moreover, the localisation of the nanoparticles can be strongly controlled during exposure to the external energy source and this represents a key element for realising a reproducible, safe and efficient *in vivo* neuromodulation method.

The further described system also provides for the possibility of using superparamagnetic nanoparticles, e.g. iron oxide and ferrites, as an active component of the matrix, in combination with an external source of an alternate magnetic field. This type of nanoparticle, in fact, when exposed to a source of alternate magnetic field, produces the conversion of the external magnetic field into heat and can thus be used for the desired neuromodulation effect, produced here by the rise in temperature.

According to a particular embodiment of the invention, the plasmonic nanoparticles are coated before being embedded in the matrix with a coating that helps to prevent uncontrolled and irreversible aggregation of the nanoparticles in the matrix itself, such as a coating with polyelectrolytes, i.e. polymers functionalised with positively or negatively charged groups such as amine or carboxyl groups, which keep the nanoparticles separated thanks to surface electrostatic repulsion. Alternatively, the coating can be made with polyethylene glycol (PEG), which, once complexed on the surface of the nanoparticles, prevents their aggregation due to steric bulk, or with any other polymeric material that facilitates their dispersion in the matrix.

According to another embodiment of this invention, in order to increase the conversion of energy from the external source into heat, a controlled aggregation of the nanoparticles can be wished for creating *hot-spots* in the matrix at the points of contact between the particles, where a strong increase in the local electric field occurs and thus a higher heat production by absorption of the external energy. In this case, the nanoparticles are not coated as described above, but used as such.

In one embodiment, the system of the invention is for use in a method for slowing, inhibiting or blocking the activity of nerves or neuronal structures in a subject in need thereof.

A further object of the present invention relates to the use of the system of the present invention for thermally or temporarily slowing, inhibiting blocking *ex vivo* the activity of a neuronal structure previously taken from a subject.

As mentioned above, further described is a medical treatment method for slowing, inhibiting or blocking the activity of nerves or neuronal structures in a subject in need thereof, comprising the steps of:
i) providing the system object comprising an injectable or implantable matrix, loaded with plasmonic or superparamagnetic nanoparticles, and an external source of NIR radiations or a source of alternate magnetic field, respectively;
ii) injecting the matrix loaded with the nanoparticles in the nerve or neuronal structure to be treated and/or on the surface thereof.
iii) placing the external energy source in a position adjacent to the neuronal structure to be treated;
iv) activating the source for energy emission long enough to slow, inhibit or block the neuronal activity in the structure.

The speed at which the matrix and the nanoparticles loaded therein are injected, also determines the distribution of the nanoparticles themselves in the tissues, which may be more or less diffused for higher or lower injection speeds, respectively.

The injection of the compound dispersed into biocompatible tissue adhesive can advantageously be carried out using double-barrelled syringes, one loaded with the fluid matrix still to be cured and the other with the curing agent, to be injected together to facilitate mixing.

The injection of the matrix loaded with the nanoparticles can be guided with the aid of *imaging* techniques combined with the addition of radioopaque or contrast agents to the matrix.

The system of this invention can be used to be surgically implanted in patients in need of a treatment to inhibit or block, permanently or temporarily, a neuronal structure; in particular embodiments, as described above, the system of this invention does not require surgical implantation, but the matrix with the nanoparticles can simply be injected and distributed in the area to be treated. This avoids resorting to traditional neuromodulation devices, which are intraneural electrodes and require invasive surgery for implantation, especially for certain parts of the nervous system that are more difficult to reach, with dissection of the nerve from the surrounding tissue to allow the device to attack the nerve and therefore also risks of damages to the surrounding tissues.

A simple syringe for the injection of the matrix allows instead the present system to be used without the need to resort to surgery, with a much smaller burden on the patient and also on health and medical personnel.

Compared to traditional intraneural electrode implants or similar devices, the present system also has the advantage of reducing the possible reactions of the organism to foreign bodies when a device is implanted in the human body, such as fibrous encapsulation or accumulation of giant cells around the implant. These phenomena are also particularly important for the efficiency of the system: encapsulation, in fact, increases the distance between electrodes and target structures and the impedance at the tissue-electrode interface, which consequently increases the amount of energy required to modulate neuronal activity. With the present system, therefore, fibrous encapsulation is reduced and with it the postoperative complications for the patient and the durability of the neuromodulatory action.

The present system reduces the invasiveness towards the patient because the external source delivers energy wirelessly, without the need for conductive wires, which would increase the risk of the body's reaction to foreign bodies, bacterial infections and breakage of conductive cables. In the present system, neuromodulation is performed by means of a photothermal effect, which transfers the energy emitted by the external source to the target nerve in the form of heat. In even minimally invasive known systems, such as injectable polymer electrodes, the presence of a filament that from the electrode reaches a subcutaneous area in the patient's body creating a conductive path is always required in order for the energy to flow preferentially in the patient's body along this path. All this is neither necessary nor useful in the present system, which therefore has for various reasons a very minimal invasiveness for the patient.

This system and the treatment method based on the use thereof in patients in need thereof, are effective in the effect of slowing, inhibiting or blocking the neuronal activity in nerves and other structures, and at the same time safe, as they can guarantee a localised and selective effect, without risk of damages to tissues and structures adjacent to the one being treated. This is also very useful because we are talking about neuronal structures, which by their nature are complex structures, in which several nerve fibres may be closely adjacent but it may be required to act on the activity of only one of the fibres or part thereof.

Finally, the system of this invention using a NIR laser source and plasmonic nanoparticles does not comprise conductive materials, therefore it has no MRI compatibility issues that are of concern in the case of patients wearing permanent neuromodulation implants.

## Claims

1. A system for thermally and temporarily slowing, inhibiting or blocking the activity of a neuronal structure, comprising:
- a matrix injectable in said neuronal structure to be treated, loaded with plasmonic nanoparticles; and
- a NIR laser source whose emissions target said matrix loaded with plasmonic nanoparticles, wherein said matrix comprises a suspension of microbeads loaded with said nanoparticles and suspended in an injectable solution.

2. The system of claim 1, wherein said plasmonic nanoparticles are nanoparticles of noble metals.

3. The system of any one of the preceding claims, wherein said nanoparticles are coated with PEG and/or with polyelectrolytes.

4. The system of any one of the preceding claims, wherein said microbeads are made with elastomers such as silicone, polyurethane, polydimethylsiloxane (PDMS), polyhydroxyalkanoates; poly(ethylen-vinyl acetate) (PEVA); elastic natural proteins such as collagen, elastin, titin, fibrillin, abductin, silk proteins, resiline; thermoresponsive gels, such as poloxamers; polymethacrylate-based copolymers; thermoplastic polyesters such as polycaprolactone (PCL), polylactic acid (PLA), polyglycolic acid (PGA) and copolymers thereof; thermosetting materials such as epoxy resins and polyimide; and lipid materials, such as waxes, fatty acids or phospholipids; and mixtures thereof.

5. The system of any of the preceding claims, wherein said matrix loaded with nanoparticles is dispersed in a suspension comprising a biocompatible adhesive material with a viscosity such that it can be injected, which suspension will solidify once injected in said neuronal structure to be treated by means of chemical or physical curing reactions, wherein the biocompatible adhesive material is selected from the group consisting of medical resins, polyoxyethylene, protein glues, such as fibrin-based glue, surgical cyanoacrylate glues, such as n-butyl-2-cyanoacrylate or 2-octyl-cyanoacrylate, and mixtures thereof.

6. The system of any one of the preceding claims, wherein said matrix loaded with nanoparticles comprises radiopaque compounds and/or air bubbles included so as to enhance the visibility of the matrix in imaging techniques.

7. The system of any of the preceding claims, further comprising at least a device for measuring the temperature in said neuronal structure and, optionally, a control unit linked to said device to which the measurement of temperature is transmitted.

8. The system of claim 7, wherein said device for measuring the temperature is a miniaturised device injectable or implantable with said matrix.

9. The system of claim 7 or 8, wherein said control unit is configured to monitor said measurement of the temperature and to control accordingly the intensity of said emissions of the source.

10. The system of any one of the preceding claims, for use in a method for thermally and temporarily slowing, inhibiting or blocking the activity of neuronal structures in a subject in need thereof.

11. Use of the system of any one of the claims 1-9, for thermally and temporarily slowing, inhibiting or blocking ex vivo the activity of a neuronal structure previously taken from a subject.

## Patentansprüche

1. System zum thermischen und vorübergehenden Verlangsamen, Hemmen oder Blockieren der Aktivität einer neuronalen Struktur, umfassend:
- eine in die zu behandelnde neuronale Struktur injizierbare Matrix, die mit plasmonischen Nanopartikeln beladen ist; und
- eine NIR-Laserquelle, deren Emissionen auf die mit plasmonischen Nanopartikeln beladene Matrix abzielen, wobei die Matrix eine Suspension von Mikrokügelchen umfasst, die mit den Nanopartikeln beladen und in einer injizierbaren Lösung suspendiert sind.

2. System nach Anspruch 1, wobei die plasmonischen Nanopartikel Nanopartikel aus Edelmetallen sind.

3. System nach einem der vorstehenden Ansprüche, wobei die Nanopartikel mit PEG und/oder mit Polyelektrolyten beschichtet sind.

4. System nach einem der vorstehenden Ansprüche, wobei die Mikrokügelchen mit Elastomeren wie Silikon, Polyurethan, Polydimethylsiloxan (PDMS), Polyhydroxyalkanoaten; Poly(ethylen-Vinylacetat) (PEVA); elastischen natürlichen Proteinen wie Kollagen, Elastin, Titin, Fibrillin, Abductin, Seidenproteinen, Resilin; thermoresponsiven Gelen, wie Poloxameren; Copolymeren auf Polymethacrylatbasis; thermoplastischen Polyestern wie Polycaprolacton (PCL), Polymilchsäure (PLA), Polyglykolsäure (PGA) und deren Copolymeren; wärmehärtenden Materialien wie Epoxidharzen und Polyimid; und Lipidmaterialien wie Wachse, Fettsäuren oder Phospholipiden; und Mischungen davon hergestellt werden.

5. System nach einem der vorstehenden Ansprüche, wobei die mit Nanopartikeln beladene Matrix in einer Suspension dispergiert ist, die ein biokompatibles Klebematerial mit einer solchen Viskosität umfasst, dass es injiziert werden kann, wobei sich die Suspension verfestigt, sobald sie in die zu behandelnde neuronale Struktur mittels chemischer oder physikalischer Aushärtungsreaktionen injiziert wird, wobei das biokompatible Klebematerial aus der Gruppe ausgewählt ist, die aus medizinischen Harzen, Polyoxyethylen, Proteinklebern, wie Kleber auf Fibrinbasis, chirurgischen Cyanoacrylatklebern, wie n-Butyl-2-Cyanoacrylat oder 2-Octyl-Cyanoacrylat, und Mischungen davon besteht.

6. System nach einem der vorstehenden Ansprüche, wobei die mit Nanopartikeln beladene Matrix röntgendichte Verbindungen und/oder eingeschlossene Luftblasen umfasst, um die Sichtbarkeit der Matrix bei bildgebenden Techniken zu verbessern.

7. System nach einem der vorstehenden Ansprüche, ferner umfassend mindestens eine Vorrichtung zum Messen der Temperatur in der neuronalen Struktur und gegebenenfalls eine mit dieser Vorrichtung verbundene Steuereinheit, an welche die Temperaturmessung übermittelt wird.

8. System nach Anspruch 7, wobei die Vorrichtung zum Messung der Temperatur eine miniaturisierte Vorrichtung ist, die mit der Matrix injizierbar oder implantierbar ist.

9. System nach Anspruch 7 oder 8, wobei die Steuereinheit konfiguriert ist, um die Temperaturmessung zu überwachen und die Intensität der Emissionen der Quelle entsprechend zu steuern.

10. System nach einem der vorstehenden Ansprüche zur Verwendung in einem Verfahren zum thermischen und vorübergehenden Verlangsamen, Hemmen oder Blockieren der Aktivität neuronaler Strukturen in einem Subjekt, das dies benötigt.

11. Verwendung des Systems nach einem der Ansprüche 1 bis 9 zum thermischen und vorübergehenden Verlangsamen, Hemmen oder Blockieren der Aktivität einer zuvor von einem Subjekt entnommenen neuronalen Struktur ex vivo.

## Revendications

1. Système permettant de ralentir, d'inhiber ou de bloquer thermiquement et temporairement l'activité d'une structure neuronale, comprenant :
- une matrice injectable dans ladite structure neuronale à traiter, chargée de nanoparticules plasmoniques ; et
- une source laser NIR dont les émissions ciblent ladite matrice chargée de nanoparticules plasmoniques, dans lequel ladite matrice comprend une suspension de microbilles chargées desdites nanoparticules et suspendues dans une solution injectable.

2. Système selon la revendication 1, dans lequel lesdites nanoparticules plasmoniques sont des nanoparticules de métaux nobles.

3. Système selon l'une quelconque des revendications précédentes, dans lequel lesdits nanoparticules sont recouvertes de PEG et/ou de polyélectrolytes.

4. Système selon l'une quelconque des revendications précédentes, dans lequel lesdites microbilles sont fabriquées avec des élastomères tels que du silicone, du polyuréthane, du polydiméthylsiloxane (PDMS), des polyhydroxyalcanoates ; du poly(éthylène-acétate de vinyle) (PEVA) ; des protéines naturelles élastiques telles que du collagène, de l'élastine, de la titine, de la fibrilline, de l'abductine, des protéines de soie, de la résiline ; des gels thermosensibles, tels que des poloxamères ; des copolymères à base de polyméthacrylate ; des polyesters thermoplastiques tels que du polycaprolactone (PCL), de l'acide polylactique (PLA), de l'acide polyglycolique (PGA) et des copolymères de ceux-ci ; des matériaux thermodurcissables tels que des résines époxy et du polyimide ; et des matières lipidiques, telles que des cires, des acides gras ou des phospholipides ; et des mélanges de ceux-ci.

5. Système selon l'une quelconque des revendications précédentes, dans lequel ladite matrice chargée de nanoparticules est dispersée dans une suspension comprenant un matériau adhésif biocompatible avec une viscosité telle qu'il peut être injecté, laquelle suspension se solidifiera une fois injectée dans ladite structure neuronale à traiter au moyen de réactions de durcissement chimiques ou physiques, dans lequel le matériau adhésif biocompatible est choisi dans le groupe constitué de résines médicales, de polyoxyéthylène, de colles protéiques, telles que des colles à base de fibrine, des colles chirurgicales à base de cyanoacrylate, telles que du n-butyl-2-cyanoacrylate ou du 2-octyl-cyanoacrylate, et des mélanges de ceux-ci.

6. Système selon l'une quelconque des revendications précédentes, dans lequel ladite matrice chargée de nanoparticules comprend des composés radio-opaques et/ou des bulles d'air afin d'améliorer la visibilité de la matrice dans les techniques d'imagerie.

7. Système selon l'une quelconque des revendications précédentes, comprenant en outre au moins un dispositif de mesure de la température dans ladite structure neuronale et, éventuellement, une unité de commande liée audit dispositif à laquelle la mesure de la température est transmise.

8. Système selon la revendication 7, dans lequel ledit dispositif de mesure de la température est un dispositif miniaturisé injectable ou implantable avec ladite matrice.

9. Système selon la revendication 7 ou 8, dans lequel l'unité de commande est configurée pour surveiller ladite mesure de la température et pour commander en conséquence l'intensité desdites émissions de la source.

10. Système selon l'une quelconque des revendications précédentes, destiné à être utilisé dans un procédé pour ralentir, inhiber ou bloquer thermiquement et temporairement l'activité de structures neuronales chez un sujet qui en a besoin.

11. Utilisation du système selon l'une quelconque des revendications 1 à 9, pour ralentir, inhiber ou bloquer thermiquement et temporairement ex vivo l'activité d'une structure neuronale préalablement prélevée sur un sujet.
